# EUROPEAN PATENT APPLICATION

(11) **EP 2 127 649 A1**
(43) Date of publication of application: **02.12.2009**
(21) Application number: 07851114.4
(22) Date of filing: 20.12.2007
(51) Int. Cl.: A61K 31/352, A23L 1/30, A61K 47/36, C08B 37/06

(54) **COMPOSITION FOR PROMOTING BIOABSORPTION OF FLAVONOID, FOOD/BEVERAGE FOR PROMOTING BIOABSORPTION OF FLAVONOID USING THE COMPOSITION, AND METHOD FOR PRODUCTION OF THE FOOD/BEVERAGE**

(30) Priority: 20.12.2006 JP 2006343164
(71) Applicant: Glico Daily Products CO., LTD, Akishima-shi, Tokyo 196-0021 (JP)
(72) Inventor: NISHIJIMA, Tomohiko, Akishima-shi Tokyo 196-0021 (JP); SAITOU, Yasuo, Akishima-shi Tokyo 196-0021 (JP); TAMURA, Shinji, Akishima-shi Tokyo 196-0021 (JP); MATSUE, Hajime, Aomori-shi Aomori 030-0947 (JP); IWAI, Kunihisa, Aomori-shi Aomori 030-0947 (JP)
(74) Representative: Laget, Jean-Loup
(86) International application number: PCT/JP2007/075226
(87) International publication number: WO 2008/075793

(57) **Abstract**

Disclosed is a flavonoid bioabsorption-promoting composition or food/beverage which can improve the bioabsorption of quercetin or other flavonoids having antioxidant effect and therefore are expected to be effective for the prevention of various diseases. It is found that ingestion of apple pectin can improve the bioabsorption of quercetin and other flavonoids by about 2 times and shows higher physiological activity compared to the case where apple pectin is not ingested. Based on this finding, a flavonoid bioabsorption-promoting composition or food/beverage can be provided, which contains apple pectin as an active ingredient and therefore can improve the bioabsorbability of a flavonoid and show a flavonoid bioabsorption-promoting activity. By using apple pectin or a food additive containing apple pectin, it becomes possible to provide a food or beverage which contains quercetin or other flavonoids, which are popularly ingested and which are classified in to a category of a processed food including a soft drink, supplement, seasoning, confectionery, and a prepared meal.

## Description

### TECHNICAL FIELD

The present invention relates to a composition capable of promoting bioabsorption of flavonoids, food or beverage capable of attaining promoted bioabsorption of flavonoids, and to a method for producing the food or beverage.

### BACKGROUND ART

A great number of flavonoids have been known in nature, which exhibit a variety of physiological activities. Flavonoids have been reported to have antioxidant, antimutagenic, anti-cancer, blood-pressure suppressive, antimicrobial/antiviral, anti-tooth decay, and anti-allergic activities. Unfortunately, however, baicalein (which is a flavonoid) has been known to be very poorly absorbed by the body tissue (only 1/300 the amount administered is absorbed) (Wakui, et al., J. Chromatog., 575, 131-136 (1992)). Also, many flavonoids, including quercetin as a typical example, are expected to exhibit preventive effects against a diversity of diseases, based on their antioxidant activity, and yet they have been found to be poorly absorbed by the body. To overcome this problem, it has been reported that when flavonoids are ingested together with lipids, absorption is slightly improved. Meanwhile, focusing on flavonoids prepared from propolis, there has been proposed a method of producing novel α-glycosyl quercetin by causing glycosyltransferase to react with a solution containing quercetin and an α-glucosyl saccharide compound. The resultant novel α-glycosyl quercetin is easily degraded in the living body through hydrolysis and exhibits inherent physiological activity of quercetin. Thus, foods, beverages, and similar products containing the thus-produced α-glycosyl quercetin have also been proposed.

The above approach requires an enzyme to react on the α-glucosyl saccharide compound, and in the pretreatment procedure, enzymatic reaction must be performed for 10 to 24 hours or thereabouts. In addition, upon completion of the enzymatic reaction, significant amounts of α-glucosyl saccharide compounds are left in the reaction mixture. Therefore, when a food product is prepared, its taste is greatly affected by such compounds. A conceivable measure to address this problem is to remove α-glucosyl saccharide compounds. However, this approach requires high cost because a step of removing α-glucosyl saccharide compounds is performed in addition to the enzymatic reaction. Therefore, in reality, it has been difficult to incorporate α-glycosyl quercetin in such an amount that shows effect in the living body. Direct use of an enzymatic reaction mixture; i.e., use of an enzymatic reaction mixture without subjecting to further treatment, will ensure a certain level of bioabsorption. However, this approach is useful only with certain flavonoids, particularly quercetin, from propolis. Quercetin is contained not only in propolis but also abundantly contained in a variety of vegetables and fruits, including onions, lettuces, broccolis, strawberries, apples, tea leaves, and buckwheat. If bioabsorption of flavonoids, including not only quercetin but also catechin (which is contained in green tea, apples, etc.) and isoflavones (which are contained in soybeans), can be ensured, a variety of foods and beverages can be provided at relatively inexpensive costs, realizing prevention of different types of diseases through physiological activities of the flavonoids. Moreover, currently the method of causing glycosyltransferase to react with a solution containing propolis-derived quercetin and an α-glucosyl saccharide compound is limited to be applied to a certain class of flavonoids which are categorized as so-called glycosides, such as a quercetin glycoside, having a sugar moiety in the molecule. Thus, those flavonoids which are categorized as aglycons, which have lost the sugar moiety, cannot be properly dealt with.
Patent Document 1: JP-A-1993-32690

### DISCLOSURE OF THE INVENTION

### PROBLEMS TO BE SOLVED BY THE INVENTION

The present invention has been achieved under the above circumstances, and to solve the mentioned problems, the invention provides a flavonoid bioabsorption-promoting composition capable of realizing promoted bioabsorption of quercetin and/or other flavonoids; foods and beverages containing the same to ensure promoted effect of flavonoid bioabsorption; and a method for producing such foods and beverages.

### MEANS TO SOLVE THE PROBLEMS

Through extensive studies, the present inventors have found that pectin has a positive effect on promoting bioabsorption of quercetin and/or other flavonoids, whereby high physiological activities of flavonoids can be ensured. Briefly, when orally ingested, flavonoids and other physiologically active substances are absorbed by the digestive tracts, and while circulating within the body via blood flow, they exhibit their pharmacological properties. In other words, in order to make effective use of the pharmacological properties of physiologically active substances within the body, the digestive tracts must take up the substances into the body. Therefore, high bioabsorption is desired for pharmacological properties to be exhibited efficiently. Flavonoids and other physiologically active substances that have been absorbed through the digestive tracts circulate via blood flow, with some fractions thereof being accumulated in organ tissue, but a major portion is ultimately discharged in urine and a minor portion is excreted in feces via bile. Therefore, concentration in blood (hereinafter referred to as blood concentration) and amount of excretion in urine are in a certain fixed relation with absorption percentage. From these perspectives, the present inventors have made careful studies with the premise that intake of pectin is achieved through the digestive tracts, focusing specifically on blood or urine samples collected from test subjects after oral administration of pectin, and have measured and evaluated the blood concentration of metabolites and %urinary excretion of metabolites. As a result, they have found the following: (1) In experiments using rats, administration of apple-derived pectin resulted in approximately twice the amount of intake of flavonoids in plasma as compared with the case of non-administration of pectin, and in similar tests on humans, improvement by about 70% was obtained from pectin administration, proving that pectin greatly improves bioabsorption; (2) Pectin concentration of lower than 0.2% does not show any contribution to bioabsorption, whereas that of 0.2% or more shows improvement in bioabsorption, and that of 0.3% or more shows significant improvement; pectin at a concentration of 3% or more does not affect bioabsorption, or even becomes unsuitable as a composition or beverage for oral consumption because pectin is excessive and acts as a thickener; and since this tendency is likely to occur at a pectin concentration of higher than 1%, pectin concentration should be 0.2 to 3%, preferably 0.3 to 1%; (3) of the two types of pectin; i.e., HM (high methoxy) pectin and LM (low methoxy) pectin, only HM pectin contributes to bioabsorption, and LM pectin does not; and (4) investigation on a great number of pectin species whose sources differ from one another revealed that, although respective pectin species were found to promote bioabsorption of flavonoids, apple-derived or citrus-derived pectin showed particularly remarkable effect.

The present invention has been achieved on the basis of the above findings, and according to the invention of claim 1, there is provided a composition capable of promoting bioabsorption of flavonoids (hereinafter referred to as flavonoid bioabsorption-promoting composition), characterized by containing pectin as an active ingredient for imparting thereto ability to promote bioabsorption of quercetin and/or other flavonoids, whereby bioabsorption of flavonoids is promoted, ensuring effective intake of flavonoids by the living body.

According to the invention of claim 2, there is provided a food or beverage capable of attaining promoted bioabsorption of flavonoids (hereinafter referred to as flavonoid bioabsorption-promoting food or beverage), characterized by containing pectin as an active ingredient for imparting thereto ability to promote bioabsorption of quercetin and/or other flavonoids, whereby bioabsorption of flavonoids is promoted, ensuring effective intake of flavonoids by the living body.

According to the invention of claim 3, there is provided a method for producing flavonoid bioabsorption-promoting food or beverage, characterized by comprising adding pectin serving as an active ingredient to food or beverage to thereby impart thereto ability to promote bioabsorption of quercetin and/or other flavonoids, whereby bioabsorption of flavonoids is promoted, ensuring effective intake of flavonoids by the living body.

The inventions of claims 4 and 5 are drawn to specific embodiments of the above-mentioned composition or food/beverage, and in these inventions, a composition or food/beverage containing pectin in preferred amounts is defined based on the findings on pectin concentration described above, and according to claim 4, there is provided a flavonoid bioabsorption-promoting composition as recited in claim 1, wherein pectin is contained in an amount of 0.2 to 3%, and according to claim 5, there is provided a flavonoid bioabsorption-promoting food or beverage as recited in claim 2, wherein pectin is contained in an amount of 0.2 to 3%.

The inventions of claims 6 and 7 are drawn to specific embodiments of the above-mentioned composition or food/beverage, and in these inventions, a composition or food/beverage exhibiting desired ability of ensuring flavonoid bioabsorption is defined based on the findings on HM pectin described above. Specifically, according to claim 6, there is provided a flavonoid bioabsorption-promoting composition as recited in claim 1 or 4, wherein pectin is an HM pectin, and according to claim 7, there is provided a flavonoid bioabsorption-promoting food or beverage as recited in claim 2 or 5, wherein pectin is an HM pectin.

The inventions of claims 8 to 10 are drawn to specific embodiments of the above-mentioned composition, food/beverage, or method for producing such food/beverage, and in these inventions, a composition, food/beverage, or method for producing the food/beverage exhibiting desired ability of ensuring flavonoid bioabsorption is defined based on the findings that the above-mentioned apple-derived or citrus-derived pectin provides desired effect. Specifically, according to claim 8, there is provided a flavonoid bioabsorption-promoting composition as recited in claim 1, 4, or 6, wherein pectin is apple-derived or citrus-derived, and according to claim 9, there is provided a flavonoid bioabsorption-promoting food or beverage as recited in claim 2, 5, or 7, wherein pectin is apple-derived or citrus-derived, and according to claim 10, there is provided a method for producing the flavonoid bioabsorption-promoting food or beverage as recited in claim 3, wherein pectin is apple-derived or citrus-derived.

The above are the gist of the present invention to solve the mentioned problems.

### EFFECT OF THE INVENTION

The present invention has the above features, and the invention of claim 1 provides a flavonoid bioabsorption-promoting composition containing pectin as an active ingredient for imparting thereto ability to promote bioabsorption of flavonoids, whereby bioabsorption of flavonoids is effectively promoted.

The invention of claim 2 provides a flavonoid bioabsorption-promoting food or beverage containing pectin as an active ingredient for imparting thereto ability to promote bioabsorption of flavonoids, whereby bioabsorption of flavonoids is effectively promoted.

The invention of claim 3 provides a method for producing flavonoid bioabsorption-promoting food or beverage containing pectin as an active ingredient for imparting thereto ability to promote bioabsorption of flavonoids, whereby bioabsorption of flavonoids is effectively promoted.

Each of the inventions of claims 4 through 9 provides a composition, or food or beverage exhibiting preferred flavonoid bioabsorption.

The invention of claim 10 provides a method for producing a food or beverage exhibiting preferred flavonoid bioabsorption.

### BRIEF DESCRIPTION OF THE DRAWINGS

Fig. 1 is a chart showing the time-course change of plasma quercetin and isorhamnetin metabolite concentration as measured after administration of quercetin.
Fig. 2 is a chart showing the amount of quercetin intake.
Fig. 3 is a chart showing quercetin metabolite concentration in plasma of blood from the aorta abdominalis (blood drawn after dissection). In FIG. 3, significant difference exists among values of respective parameters which do not share the same notation.
Fig. 4 is a set of charts showing plasma quercetin metabolite concentrations in the caudal artery.
Fig. 5 is a chart showing cumulative percentage of excretion in urine of administered quercetin.
Fig. 6 is a chart showing plasma quercetin metabolite concentrations in the caudal artery, as measured after administration of test feeds containing pectin at different concentrations.
Fig. 7 is a chart showing plasma quercetin metabolite concentrations in the caudal artery, as measured after administration of test feeds containing different types of pectin.
Fig. 8 is a chart showing % absorption when quercetin is ingested via beverages containing 0% pectin or 1% pectin.
Fig. 9 is a chart showing percentage of quercetin excretion in urine.

### BEST MODE FOR CARRYING OUT THE INVENTION

The present invention will next be described in more detail. The flavonoid bioabsorption-promoting composition of the present invention contains pectin as an active ingredient, whereby ability to promote bioabsorption of quercetin or other flavonoids is imparted to the composition; the flavonoid bioabsorption-promoting food or beverage contains pectin as an active ingredient, whereby ability to promote bioabsorption of quercetin or other flavonoids is imparted to the food or beverage; and the method for producing flavonoid bioabsorption-promoting food or beverage comprises adding pectin serving as an active ingredient to food or beverage to thereby impart thereto ability to promote bioabsorption of quercetin and/or other flavonoids. According to the present invention, the mentioned composition, food, and beverage are intended to be consumed orally by humans, whereby flavonoid bioabsorption can be exhibited effectively.

When used in the above manner, pectin exhibits high physiological activity and positively affects bioabsorption of quercetin and/or other flavonoids. However, experiments have confirmed that a pectin content of 0.1% cannot produce this effect, and significant absorption promoting effect can be obtained when pectin content is 0.3% to 1%, 3%, or 5%. Also, criticality in terms of absorption promoting effect was found to exist between 0.1% and 0.3% of pectin content. Accordingly, the lower limit of pectin content that manifests the absorption promoting effect is presumed to be around 0.2%. Thus, the lower limit of pectin content is 0.2% or more, preferably 0.3% or more. When pectin content is increased, on one hand, flavonoid bioabsorption effect is enhanced, and on the other hand, increased thickness or even coagulation may result. This is because pectin has viscosity-increasing or coagulation-inducing property, as is known to be used as a thickener, gelling agent, or a similar agent. Thus, pectin content of higher than 3% will impede taste and texture flavor due to increased viscosity or coagulation, making the composition, food, or beverage unsuitable for oral consumption, and tendency of this unfavorable phenomenon may occur when pectin content is higher than 1%. Therefore, the upper limit of pectin content is 3% or less, preferably 1% or less.

Based on the DE value (i.e., methyl esterification degree) which indicates percentage of methyl galacturonate present in the entire galacturonide units, pectin is generally divided into HM (high-methoxyl) pectin (DE ≥ 50%) and LM (low-methoxyl) pectin (DE < 50%). Through experiments, bioabsorption promoting effect of pectin was found to be attributed to one of these two types of pectin, HM pectin. Thus, preferably, HM pectin, namely, pectin having a DE value of 50% or more is employed.

Since pectin, a natural high-molecular polysaccharide is linked with cellulose, etc. as a component of the cell wall of fruit and vegetables, the origin of pectin is considered not to be particularly limited. Through experiments, however, it has been found that apple-derived pectin and citrus-derived pectin are effective in enhancing and promoting bioabsorption of flavonoids, while providing ensured bioabsorption effect. Therefore, pectin is preferably an apple-derived pectin or citrus-derived pectin. When pectin is so selected, there can be easily and reliably obtained a composition, food, or beverage that effectively exhibits flavonoid bioabsorption effect.

Apple-derived pectin is obtained from the apple, which is fruit of a fruit tree belonging to the rose family Rosaceae, and citrus-derived pectin is obtained from *C. unshu,* which is fruit of a fruit tree belonging to Rutaceae. It is considered that no particular limitation should be imposed on the variety or improved variety of respective plants. These two types of pectin can be obtained, for example, by reacting acid or alkali with apple protopectin or citrus protopectin. Generally, commercially available apple pectin or citrus pectin may be conveniently employed to obtain effective flavonoid bioabsorption promoting action. Moreover, although pectin typically occurs in polymer form, it is envisaged that lower-molecular pectin, which is generated through breakdown of pectin by a degrading enzyme such as pectinase or pectate lyase, as well as oligosaccharides are also useful.

A flavonoid bioabsorption-promoting composition to which an active ingredient apple pectin or citrus pectin has been incorporated with an aim to provide flavonoid bioabsorption effect may be processed into a food additive to be added to a flavonoid-containing food. The food additive may take the form of solution, powder, or any other suitable form. Alternatively, the flavonoid bioabsorption-promoting composition may be combined with a flavonoid to prepare a food additive to be added to other types of foodstuff. Thus, the composition of the present invention may be put on the market as a food additive. Also, flavonoid bioabsorption-promoting foods and beverages may be put on the market in various product forms including soft drinks; supplements; flavorings; confectionery such as chocolate, cookies, biscuits, gummies, gums, jellies; processed foods such as ice creams, sherbets, yoghurt, cheese, curry, retort pouch foods, and Chinese noodle; and other everyday foods and beverages which are ordinarily consumed, including ham, sausages, and other delicatessens, wherein all these items contain quercetin or other flavonoids along with apple pectin or citrus pectin *per se*, or the above-mentioned food additives containing apple pectin or citrus pectin. In order to produce such flavonoid bioabsorption-promoting foods and beverages, pectin *per se*; e.g., apple pectin or citrus pectin, or any of the above-mentioned food additives containing such pectin is employed as a raw material to be handled in the manufacturing factory. Alternatively, it may be added to the intermediate stage of manufacture. In the case of the manufacture of flavonoid bioabsorption-promoting compositions, similar approaches are taken.

In this connection, when apple pectin or citrus pectin is incorporated into food or beverage, the pectin may be employed together with food additives which are added to the food or beverages, and choice of such food additives is determined depending on the nature of the composition, food, or beverage.

Flavonoids, whose absorption is promoted by an active ingredient pectin (e.g., the mentioned apple pectin or citrus pectin), include not only quercetin but also catechin which is contained in green tea, apple, etc., and isoflavons contained in soybeans. Thus, examples of food and beverages to which ability to promote bioabsorption of flavonoids is imparted include, but are not limited to, soft drinks such as green tea beverages, vegetable- or fruit-based beverages, and fruit juices; supplements produced using vegetables or fruit; and other types of processed food. These are particularly preferred as they contain flavonoids in great quantity and thus amply exhibit apple pectin's flavonoid-bioabsorption promoting action. Therefore, incorporation of pectin into the mentioned green tea beverages, vegetable- or fruit-based beverages, fruit juices, etc. which contain flavonoids is a recommended means for ingestion of pectin. In this connection, pectin's effect of promoting flavonoid bioabsorption is manifested by any of the following two modes: oral ingestion of a preparation containing both the pectin and flavonoid (simultaneous intake), and oral ingestion of pectin and flavonoid separately (each ingested solely). Thus, either mode will produce the effect of pectin.

Also, pectin's effect of promoting flavonoid bioabsorption is manifested both by single dosage and by continued dosage. Therefore, for ensuring promoted bioabsorption of flavonoids, continued ingestion of the composition, food, or beverage of the present invention is not necessarily essential. That is, through experiments, pectin's effect of promoting flavonoid bioabsorption has been confirmed not only for continued intake but also on the exact day of ingestion. This indicates that a single dosage is also useful.

Examples of flavonoids include substances belonging to vitamin P, such as hesperidin and rutin; diosmin; and naringin which causes bitterness. These materials are known to have vasodepressor activity, cholesterol-lowering effect, anti-cancer activity, or diabetes preventive effect. As used herein, the term "flavonoid(s)" encompasses not only typical flavonoids such as flavonols, flavanones, flavanonols, flavans, flavones, flavanols, anthocyanidins, and chalcones, but also isoflavones. The flavonoids may be in the form of glycosides or aglycons (having lost the sugar moiety). Examples of flavonoids include eriocitrin, neoeriocitrin, narirutin, naringin, hesperidin, hesperetin, neohesperidin, neoponcirin, poncirin, rutin, quercetin, isorhoifolin, rhoifolin, diosmin, neodiosmin, sinensetin, nobiletin,.tangeritin, catechin, catechin gallate, epigallocatechin, epigallocatechin gallate, oolong tea polymerized polyphenol, anthocyanin, and heptamethoxyflavone. In the present invention, since flavonoid is mixed with pectin, flavonoid may be used solely, or alternatively, two or more flavonoids may be used as a mixture. Moreover, the flavonoid may be a glycoside or an aglycon.

### Example 1

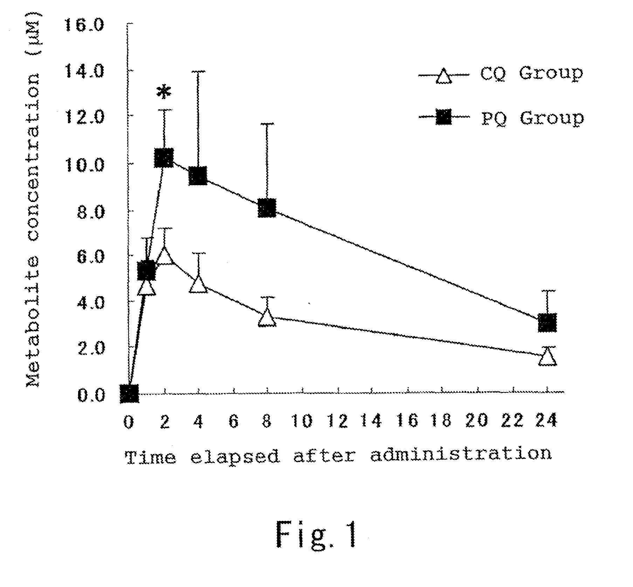

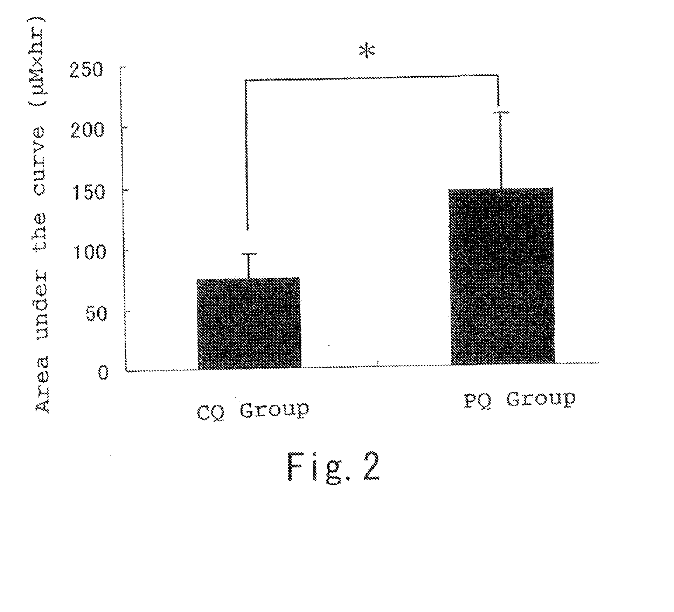

Wistar male rats (7 weeks old) were preliminarily reared for one week on a standard solid diet (MF, product of Oriental Yeast Co., Ltd.). The rats were divided into 2 groups, each consisting of 5 rats, so that the two groups had the same average body weight. To one of the 2 groups, PQ group (consisting of 5 rats), test diet was given ad libitum for 42 days. The diet contained pectin (apple-derived pectin; product of Sigma Aldrich Japan) in an amount of 5% w/w as a replacement of cellulose (see Table 1, column PQ). On day 43, the rats were fasted for 17 hours, and then orally given a quercetin suspension (50mg of quercetin/kg body weight/3 mL) prepared by suspending quercetin and 1% w/w carboxymethylcellulose-Na in distilled water via stomach tube. Following 0, 1, 2, 4, 8, and 24 hours of administration, blood was drawn from the caudal artery of each rat, to thereby collect plasma samples from the 5 rats of the PQ group. The amount of plasma quercetin was determined as follows. Deconjugation reaction was allowed to proceed for 120 minutes using β-glucuronidase, followed by extraction with acetone. The extract was brought to dryness under reduced pressure. The dry solid was dissolved in a solvent (0.5% aqueous phosphoric acid solution : acetonitrile = 65 : 35), and the resultant solution was subjected to measurement by means of HPLC-UV (370 nm). The total amount of quercetin and isorhamnetin metabolites was taken as plasma quercetin, and was plotted on a concentration-time curve. Amount of intake (absorption) up to 24 hours following administration was calculated as the area under the concentration-time curve (AUC, µM×hr). The statistically significant test employed was Student's t-test. Data showing a significant difference at a level of significance of < 5% are marked with an asterisk. The results are shown in Figs. 1 and 2.

**Table 1**

| Composition of test diet (g/100g) | | |
|---|---|---|
| | CQ | PQ |
| Casein | 20.0 | 20.0 |
| α-Cornstarch | 13.0 | 13.0 |
| β-Cornstarch | 40.5 | 40.5 |
| Sucrose | 10.0 | 10.0 |
| Corn oil | 7.0 | 7.0 |
| Blended with mineral AIN-93G | 3.5 | 3.5 |
| Blended with vitamin AIN-93 | 1.0 | 1.0 |
| Cellulose powder | 5.0 | 0.0 |
| Apple pectin | 0.0 | 5.0 |

### Comparative Example 1

The procedure of Example 1 was repeated except that the 5 rats of the other group of the 2 groups; i.e., CQ group, were given a test diet that had been prepared to have a pectin content of 0% w/w (see Table 1, column CQ). The results are shown in Figs. 1 and 2.

When the results obtained from Example 1 are compared with those of Comparative Example 1, the 5 members of the PQ group, which took a diet containing 5% w/w apple pectin, exhibited approximately twice the amount of absorbed quercetin as measured in Comparative Example 1 (diet containing no pectin), confirming that apple pectin has effect of promoting bioabsorption of quercetin; i.e., flavonoid bioabsorption promoting effect.

### Example 2

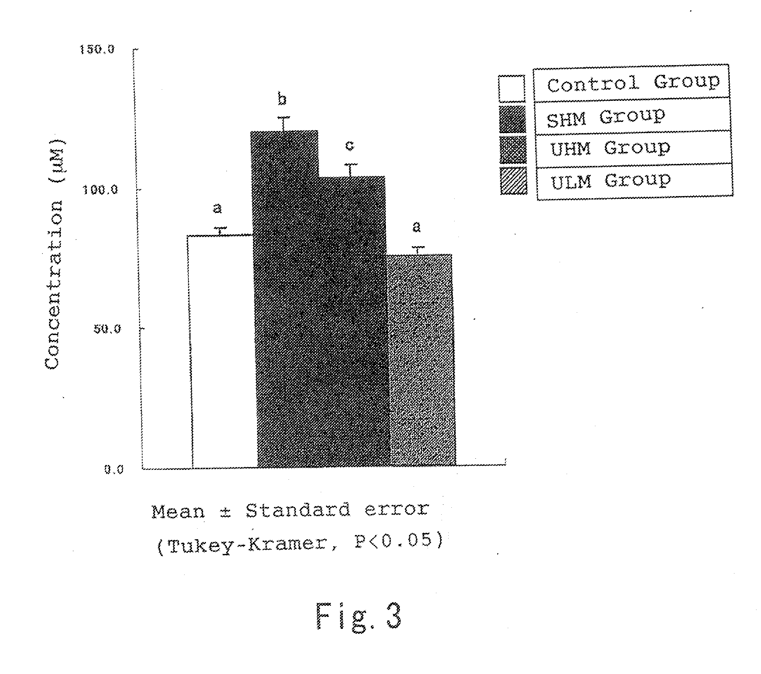

Twenty four Wistar male rats (7 weeks old) were divided into 4 groups, each consisting of 6 rats. The 6 rats of the SHM group and the 6 rats of the UHM group were given test diets respectively and were allowed to consume them ad libitum. The test diets contained HM pectin (apple-derived pectin; product of Sigma Aldrich Japan and UNIPECTIN HM-1, product of Unitec Foods Co., Ltd.) in an amount of 5% w/w as a replacement of cellulose (see Table 2, columns for pectin administration groups). On day 21 of administration, blood was drawn from the abdominal aorta and plasma samples were obtained. In a manner similar to that in Example 1, the amount of plasma quercetin metabolites was determined by means of HPLC-UV. The results are shown in Fig. 3.

**Table 2**

| Composition of test diet (%) | | | | |
|---|---|---|---|---|
| Composition | Control group | SHM group | UHM group | ULM group |
| Casein | 20.0 | 20.0 | 20.0 | 20.0 |
| α-Cornstarch | 13.0 | 13.0 | 13.0 | 13.0 |
| β-Cornstarch | 40.3 | 40.3 | 40.3 | 40.3 |
| Sucrose | 10.0 | 10.0 | 10.0 | 7.8 |
| Corn oil | 7.0 | 7.0 | 7.0 | 7.0 |
| Blended with mineral (AIN-93G) | 3.5 | 3.5 | 3.5 | 3.5 |
| Blended with vitamin (AIN-93) | 1.0 | 1.0 | 1.0 | 1.0 |
| Cellulose | 5.0 | - | - | - |
| Pectin of Sigma Corp. | - | 5.0 | - | - |
| HM pectin | - | - | 5.0 | - |
| LM pectin | - | - | - | 5.0 |
| Quercetin | 0.2 | 0.2 | 0.2 | 0.2 |

### Comparative Example 2

The procedure of Example 2 was repeated except that the 6 rats of the control group in the mentioned 4 groups were given a test diet that had been prepared to have a pectin content of 0% w/w (see Table 2, the column for control group), and the 6 rats of the ULM group were similarly given a test diet containing LM pectin (apple-derived pectin, UNIPECTIN LMSN325, product of Unitec Foods Co., Ltd.) in an amount of 5% w/w as a replacement of cellulose (see Table 2, the column for ULM group). The results are also shown in Fig. 3.

When the results of Example 2 are compared with those of Comparative Example 2, the SHM and UHM groups exhibited significantly higher values of plasma quercetin metabolite content over corresponding values from the control group in which pectin was not administered, and from the ULM group in which LM pectin was administered. Thus, apple pectin was found to enhance absorption of quercetin, when administered simultaneously therewith. In addition, apple pectin was also found to exhibit effect on HM pectin having a DE value of 50% or more, but no effect on LM pectin. Based on these findings, the methyl group of the pectin molecule presumably takes part in the mechanism by which apple pectin promotes bioabsorption of quercetin.

### Example 3

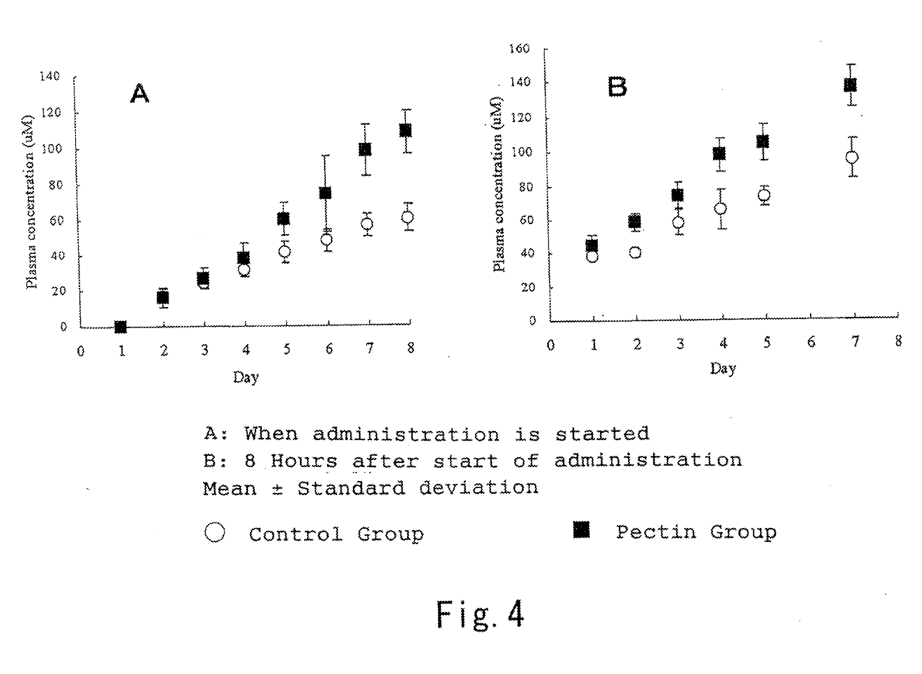

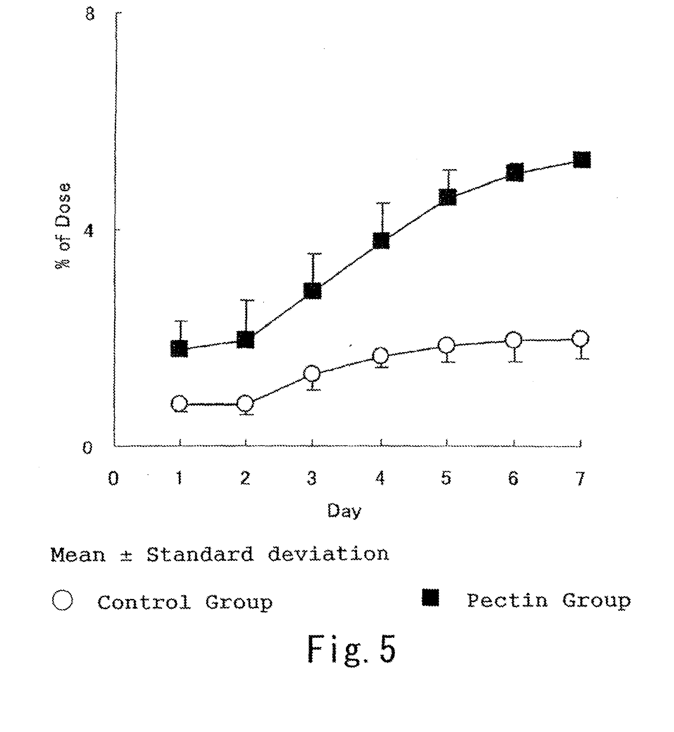

Twelve Wistar male rats (7 weeks old) were divided into 2 groups, each consisting of 6 rats. The 6 rats of one group, to which pectin is administered (pectin group), were reared on a restricted feeding. Specifically, the rats were given a test diet containing 0.2% w/w quercetin and 5% w/w apple pectin 8 hours a day for 7 days (see Table 3, column for pectin group). Every day, before and after the period of test diet feeding, caudal artery plasma and 24-hour urine after ingestion were collected. At the start of administration and at the point after 8 hours from the start of administration, amount of quercetin metabolites in plasma and urine were determined through HPLC-UV. During the period of feeding the test diets, the two groups were mutually equivalent in terms of body weight and amount of diet consumption. Moreover, the quercetin amount consumed within the daily 8 hours of feeding remained the same from test day to test day. The results obtained at the start of administration and those obtained after 8 hours are shown in Fig. 4, and data of cumulative excretion (%) in urea of administered quercetin are shown in Fig. 5.

**Table 3**

| Composition of test diet (%) | | |
|---|---|---|
| Composition | Control group | Pectin group |
| Casein | 20.0 | 20.0 |
| α-Cornstarch | 13.0 | 13.0 |
| β-Cornstarch | 40.3 | 40.3 |
| Sucrose | 10.0 | 10.0 |
| Corn oil | 7.0 | 7.0 |
| Blended with mineral (AIN-93G) | 3.5 | 3.5 |
| Blended with vitamin (AIN-93) | 1.0 | 1.0 |
| Cellulose | 5.0 | - |
| Apple pectin | - | 5.0 |
| Quercetin | 0.2 | 0.2 |

### Comparative Example 3

The procedure of Example 3 was repeated except that the control group in the mentioned 2 groups was given a test diet that had been prepared to contain 0.2% w/w quercetin and 5% w/w cellulose (see Table 3, the column for control group). The results are shown in Figs. 4 and 5.

When the results of Example 3 are compared with those of Comparative Example 3, on day 1 (8 hours after start of administration), plasma concentration of metabolites in Example 3 was significantly higher than its counterpart concentration. Also, on day 1 (24 hours after start of administration), %excretion of metabolites in urine was also significantly high. The plasma concentration of metabolites and %excretion of the metabolites in urine both increased as the ingestion days accumulated, thereby expanding the difference against those obtained from the control group of Comparative Example 3. These findings suggest that the effect of improving absorption of quercetin caused by apple pectin which was simultaneously ingested is not attributed to any change in small intestinal environment or a similar mechanism which requires time before the effect is manifested, but is an instantaneous effect, as proven by the fact that a significant difference was confirmed even for a single time of ingestion.

### Example 4

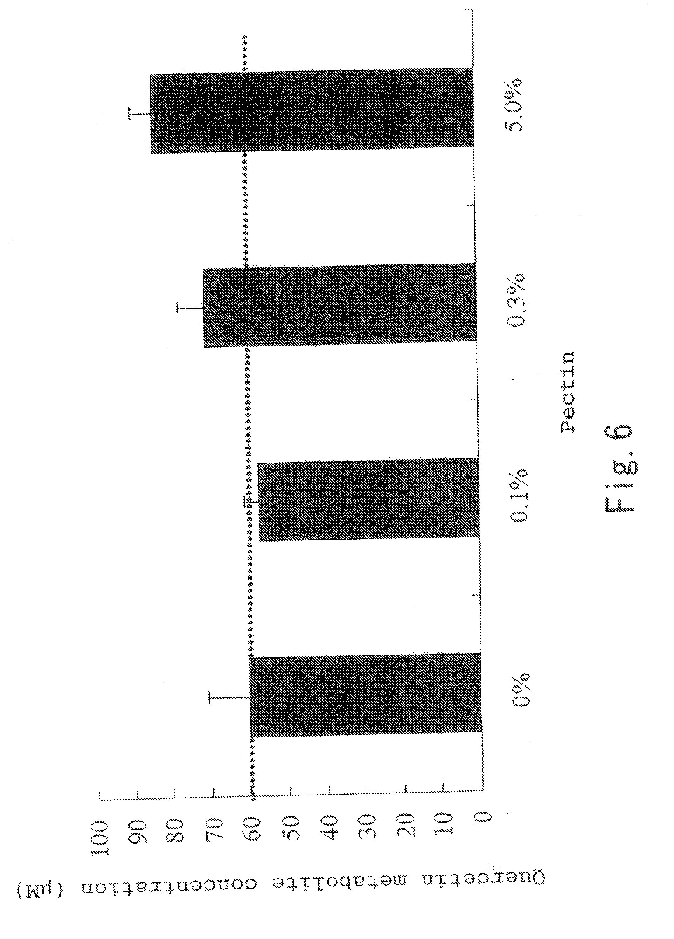

Twelve Wistar male rats (7 weeks old) were divided into 4 groups, each consisting of 3 rats. Test diets were prepared based on a concept of partially or entirely replacing the amount of cellulose with apple pectin. The rats belonging to the pectin 0.3% group and pectin 5.0% group were reared on a restricted feeding. Specifically, the rats were given a 0.3% pectin test diet containing 0.3% w/w apple pectin and 0.2% w/w quercetin (for pectin 0.3% group) or a 5.0% pectin test diet containing 5.0% w/w apple pectin and 0.2% w/w quercetin (for pectin 5.0% group) (see Table 4, pectin 0.3% group and pectin 5.0% group) from 9 a.m. to 5 p.m. (8 hours) every day for 3 days. Before and after the period of test diet feeding; i.e., at 9 a.m. and 5 p.m., blood was drawn from the caudal artery, and plasma samples were obtained. The amount of plasma quercetin metabolites in each rat was determined through HPLC-UV. During the period of feeding the test diets, the two groups were mutually equivalent in terms of body weight and amount of diet consumption. Moreover, the quercetin amount consumed within the daily 8 hours of feeding remained the same from test day to test day. Fig. 6 shows the measurements of quercetin metabolite concentration obtained when the first 56 hours had elapsed after starting of the test (i.e., at 5 p.m. on day 3 of ingestion), when the difference between the two groups became the most apparent.

**Table 4**

| Composition of test diet (%) | | | | |
|---|---|---|---|---|
| | Pectin | Pectin | Pectin | Pectin |
| Composition | 0% | 0.1% | 0.3% | 5.0% |
| | group | group | group | group |
| Casein | 20.0 | 20.0 | 20.0 | 20.0 |
| α-Cornstarch | 13.0 | 13.0 | 13.0 | 13.0 |
| β-Cornstarch | 40.3 | 40.3 | 40.3 | 40.3 |
| Sucrose | 10.0 | 10.0 | 10.0 | 10.0 |
| Corn oil | 7.0 | 7.0 | 7.0 | 7.0 |
| Blended with mineral (AIN-93G) | 3.5 | 3.5 | 3.5 | 3.5 |
| Blended with vitamin (AIN-93) | 1.0 | 1.0 | 1.0 | 1.0 |
| Cellulose | 5.0 | 4.9 | 4.7 | - |
| Apple pectin | - | 0.1 | 0.3 | 5.0 |
| Quercetin | 0.2 | 0.2 | 0.2 | 0.2 |

### Comparative Example 4

The procedure of Example 4 was repeated except that, of the mentioned four groups, a pectin 0% group and pectin 0.1% group were respectively given a 0% pectin test diet containing 0% w/w apple pectin and 0.2% w/w quercetin (for pectin 0% group) or a 0.1% pectin test diet containing 0.1% w/w apple pectin and 0.2% w/w quercetin (for pectin 0.1% group) (see Table 4, pectin 0% group and pectin 0.1% group). The results are shown in Fig. 6.

When the results of Example 4 are compared with those of Comparative Example 4, during the period of feeding the test diets, in the pectin 0.3% group and pectin 5.0% group, quercetin metabolite concentrations in the plasma samples obtained from the caudal artery stayed higher than those in the pectin 0% group and pectin 0.1% group tested in Comparative Example 4. From these data, it is concluded that criticality in terms of quercetin absorption promoting effect was found to exist between 0.1% and 0.3% of pectin content, and its lower limit is approximately 0.2%. Thus, the lower limit of apple pectin intake that is required for obtaining quercetin absorption promoting effect is concluded to be 0.2% or more, preferably 0.3% or more.

### Example 5

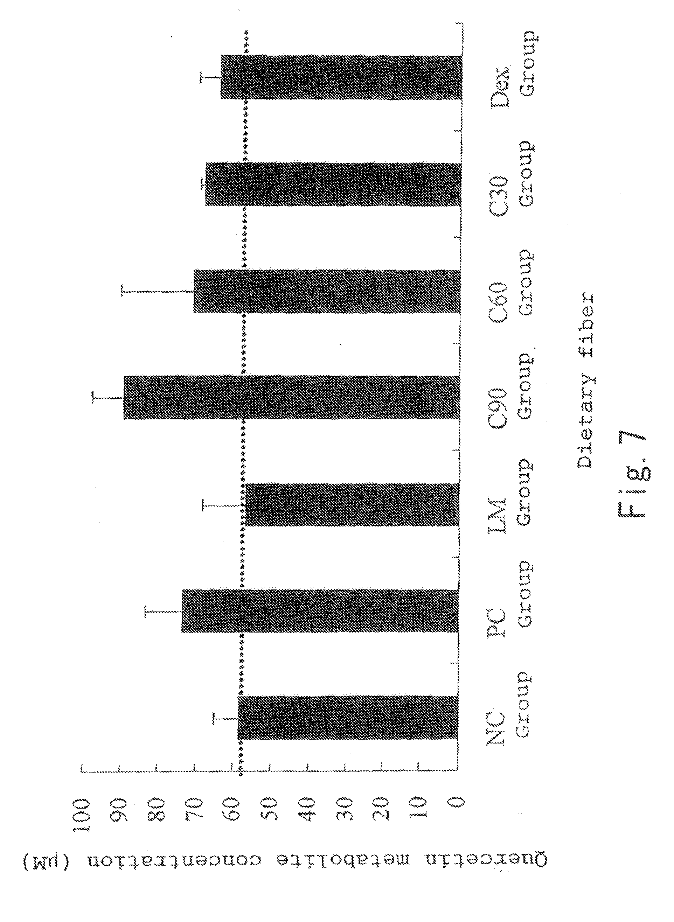

Twenty-one Wistar male rats (7 weeks old) were divided into 7 groups, each consisting of 3 rats. Test diets were prepared based on a concept of replacing dietary fiber with 5% w/w pectin and including 0.2% w/w quercetin, and the resultant diets were given to the rats belonging to the three groups in the mentioned 7 groups; i.e., PC group, C90 group, and C60 group. The procedure of Example 4 were repeated except that blended diets PC (HM pectin, apple-derived product of Sigma Aldrich Japan), C90 (pectin having an esterification degree of 90%, citrus-derived product of Sigma Aldrich Japan), and C60 (pectin having an esterification degree of 60%, citrus-derived product of Sigma Aldrich Japan) were given to the rats (see Table 5). During the period of feeding the test diets, rat groups were mutually equivalent in terms of body weight and amount of diet consumption. Moreover, the quercetin amount consumed within the daily 8 hours of feeding remained the same from test day to test day. Fig. 7 shows the measurements of quercetin metabolite concentration obtained when the first 56 hours had elapsed after starting of the test (i.e., at 5 p.m. on day 3 of ingestion), when the inter-group differences became the most apparent.
(Left blank)

**Table 5**

| Composition of test diet (%) | | | | | | | |
|---|---|---|---|---|---|---|---|
| Composition | NC | PC | LM | C90 | C60 | C30 | Dex |
| Casein | 20.0 | 20.0 | 20.0 | 20.0 | 20.0 | 20.0 | 20.0 |
| α-Cornstarch | 13.0 | 13.0 | 13.0 | 13.0 | 13.0 | 13.0 | 13.0 |
| β-Cornstarch | 40.3 | 40.3 | 40.3 | 40.3 | 40.3 | 40.3 | 40.3 |
| Sucrose | 10.0 | 10.0 | 10.0 | 10.0 | 10.0 | 10.0 | 10.0 |
| Corn oil | 7.0 | 7.0 | 7.0 | 7.0 | 7.0 | 7.0 | 7.0 |
| Blended with mineral (AIN-93G) | 3.5 | 3.5 | 3.5 | 3.5 | 3.5 | 3.5 | 3.5 |
| Blended with vitamin (AIN-93) | 1.0 | 1.0 | 1.0 | 1.0 | 1.0 | 1.0 | 1.0 |
| Cellulose | 5.0 | - | - | - | - | - | - |
| Apple HM pectin | - | 5.0 | - | - | - | - | - |
| Apple LM pectin | - | - | 5.0 | - | - | - | - |
| Citrus pectin | - | - | - | - | - | - | - |
| (degree of esterification: 90%) | - | - | - | 5.0 | - | - | - |
| Citrus pectin | - | - | - | - | - | - | - |
| (degree of esterification: 60%) | - | - | - | - | 5.0 | - | - |
| Citrus pectin | - | - | - | - | - | - | - |
| (degree of esterification: 30%) | - | - | - | - | - | 5.0 | - |
| Indigestible dextrin | - | - | - | - | - | - | 5.0 |
| Quercetin | 0.2 | 0.2 | 0.2 | 0.2 | 0.2 | 0.2 | 0.2 |

### Comparative Example 5

The procedure of Example 5 was repeated except that the NC group was given a test diet NC containing 0% w/w apple pectin and 5% cellulose (product of Oriental Yeast Co., Ltd.); the LM group was given a test diet containing 5% w/w apple-derived LM pectin (UNIPECTIN LM SN325, product of Unitec Foods Co., Ltd.) for replacing cellulose; the C30 group was given a C30 test diet containing 5% w/w citrus-derived pectin (citrus-derived pectin having an esterification degree of 30%, product of Sigma Aldrich Japan); and the Dex group was given a test diet Dex containing 5% w/w indigestible dextrin (product of Matsutani Kagaku). All the diets further contained 0.2% w/w quercetin. The results are shown in Fig. 7.

When the results of Example 5 are compared with those of Comparative Example 5, the PC group in which apple HM pectin was employed, and the C90 and C60 groups in which citrus HM pectin was employed respectively exhibited higher values than counterpart measurements of Comparative Example 5. Thus, apple-derived and citrus-derived HM pectins both produced a significant effect of promoting quercetin absorption, as compared with the LM pectin or dietary fiber tested in Comparative Example 5.

### Example 6

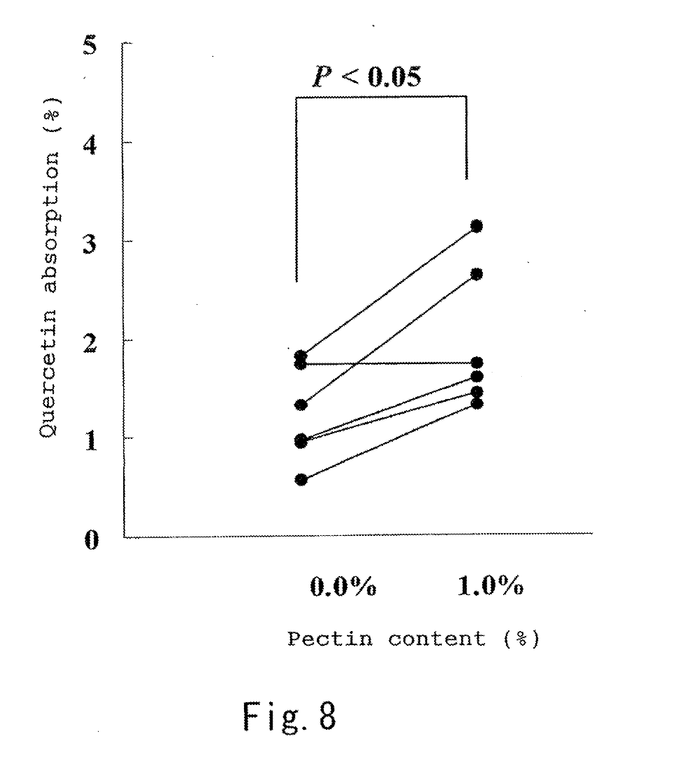

Six adult men in their twenties to thirties (mean age: 32.2±2.5; mean body weight: 72.5±8.3 kg, BMI: 23.6±2.9) participated in the human sample collection test. Three persons before undergoing the test cycle of Comparative Example 6 (will be described herein later) and three persons after they had completed the test cycle of Comparative Example 6 (in this case, the test was conducted 2 or more days after completion of the test cycle) participated in the test. Briefly, quercetin aglycon (70 mg) and 1% w/w apple pectin were suspended in a test beverage P (200 mL) in Table 6, and the test subjects ingested the resultant suspension. The total urine excreted during the 24 hours following ingestion was recovered, and the amount of quercetin metabolites contained in the urine was quantitatively determined by HPLC-UV, whereby the amount of absorption was assessed. The test subjects were requested to avoid any food containing vegetable polyphenols or fruit polyphenols from the evening meal on one day before the injection day. On the test day, consumption of any items other than water was prohibited. At 10 a.m., the test subjects were requested to drink 200 mL of a test beverage and at each discharge of urine, the entire volume of the discharged urine was collected in a 250-mL container (125 mg of ascorbic acid was previously added thereto). After the weight of the discharged urine was measured, a portion of the urine was taken and stored at -80°C. The urine discharged after the subject had back to home was pooled in a 1-L plastic bottle to which 500 mg ascorbic acid had been added. The bottle was collected next morning between 9:30 a.m. and 10 a.m. Subsequently, as the final collection, urine was again collected at around 10 a.m., whereby the urine collection test was completed. On the urine collection day, lunch meal was prepared by a person in charge of the test. All the test subjects had a meal of the same menu (10 pieces of *nigirisushi* and one rice ball) at the same time. Mineral water (1 L) was also allotted to each person, and the participants were requested to drink the entire volume between 10 a.m. and 6 p.m. Urine quercetin metabolite content of each of the collected urine samples was determined by HPLC-UV, and through calculation based on quercetin aglycon, the amount of excretion was obtained. From the percent absorption (%, (volume of excretion) / (volume of intake)) calculated from the cumulative amount of excretion during 24 hours of ingestion, performance of pectin-containing beverages on quercetin absorption was evaluated. The results are shown in Fig. 8.

**Table 6**

| Compositions of 0% pectin beverage (W) and 1% pectin beverage (P) | | |
|---|---|---|
| Composition | Test beverage W | Test beverage P |
| Pectin | 0.00% | 1.00% |
| Quercetin | 0.05% | 0.05% |
| Sugar | 7.50% | 7.50% |
| Tartaric acid | 0.13% | 0.13% |
| Flavor | 0.10% | 0.10% |
| Water | 92.23% | 91.23% |
| Total | 100.00% | 100.00% |

### Comparative Example 6

Of the mentioned 6 test subjects, 3 subjects prior to undergoing the test cycle of Example 6 and 3 subjects after finishing the test (an interval of 2 or more days was placed after the completion of the test) participated in this test. The procedure of Example 6 was repeated except that a test beverage W in Table 6, which contained no apple pectin, was employed. The results are shown in Table 8.

When the results of Example 6 are compared with those of Comparative Example 6, in the case where quercetin was ingested as contained in beverage P, percentage excretion of quercetin metabolites in urine; i.e., percent quercetin absorption, was found to have improved by approximately 70% on average (P<0.05, paired t-test), thus confirming that pectin exhibits its meritorious effect of promoting quercetin absorption in humans.

### Example 7

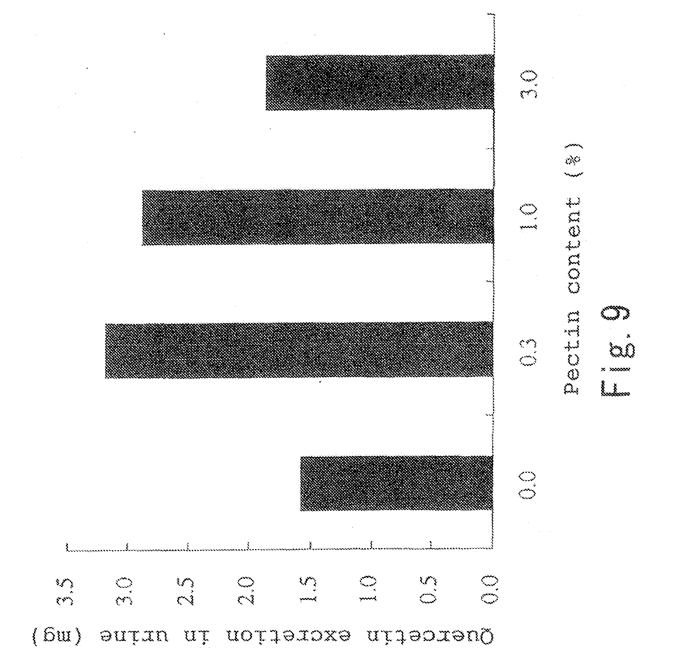

Two adult men, one in his twenties and the other in his thirties, were requested to drink a test beverage (200 mL, suspension) containing quercetin aglycon (70 mg) and 0.3% w/w, 1% w/w, or 3% w/w apple pectin (see Table 7). The general procedure of Example 6 was followed, and the amount of quercetin metabolites was determined by HPLC-UV, whereby the excretion volume was evaluated. The results are shown in Fig. 9.

**Table 7**

| Compositions of beverages of different pectin concentrations | | | | |
|---|---|---|---|---|
| Composition | 0% | 0.3% | 1% | 3.0% |
| Pectin | 0.00% | 0.30% | 1.00% | 3.00% |
| Quercetin | 0.05% | 0.05% | 0.05% | 0.05% |
| Sugar | 7.50% | 7.50% | 7.50% | 7.50% |
| Tartaric acid | 0.13% | 0.13% | 0.13% | 0.13% |
| Flavor | 0.10% | 0.10% | 0.10% | 0.10% |
| Water | 92.23% | 91.93% | 91.23% | 89.23% |
| Total | 100.00% | 100.00% | 100.00% | 100.00% |

The general procedure of Example 7 was repeated, except that the test beverage containing no pectin was employed (see Table 7, the column of 0% w/w). The results are also shown in Fig. 9.

When the results of Example 7 are compared with those of Comparative Example 7, ingestion of test beverages containing apple pectin in an amount of 0.3% w/w, 1% w/w, or 3% w/w enhanced the amount of excretion of quercetin metabolites in urine over the case of 0% w/w apple pectin. Thus, in order to obtain the effect of promoting quercetin absorption, the amount of apple pectin should be 0.2% or more, preferably, 0.3% or more. In this connection, since consumption of a pectin-containing beverage is equivalent to consumption of one apple (200 g; pectin content = 0.6 g, according to "Standard Tables of Food Composition in Japan," fifth revised edition) in terms of pectin intake and concentration, by incorporating pectin at a higher amount than contained in apple will provide effect of promoting bioabsorption of flavonoids.

## Claims

1. A flavonoid bioabsorption-promoting composition, **characterized by** containing pectin as an active ingredient for imparting thereto ability to promote bioabsorption of quercetin or other flavonoids.

2. A flavonoid bioabsorption-promoting food or beverage, **characterized by** containing pectin as an active ingredient for imparting thereto ability to promote bioabsorption of quercetin or other flavonoids.

3. A method for producing flavonoid bioabsorption-promoting food or beverage, **characterized by** comprising the step of adding pectin serving as an active ingredient to food or beverage to thereby impart thereto ability to promote bioabsorption of quercetin or other flavonoids.

4. The flavonoid bioabsorption-promoting composition as recited in claim 1, wherein pectin is contained in an amount of 0.2 to 3%.

5. The flavonoid bioabsorption-promoting food or beverage as recited in claim 2, wherein pectin is contained in an amount of 0.2 to 3%.

6. The flavonoid bioabsorption-promoting composition as recited in claim 1 or 4, wherein pectin is HM pectin.

7. The flavonoid bioabsorption-promoting food or beverage as recited in claim 2 or 5, wherein pectin is HM pectin.

8. The flavonoid bioabsorption-promoting composition as recited in claim 1, 4, or 6, wherein pectin is apple-derived or citrus-derived pectin.

9. The flavonoid bioabsorption-promoting food or beverage as recited in claim 2, 5, or 7, wherein pectin is apple-derived or citrus-derived pectin.

10. The method for producing the flavonoid bioabsorption-promoting food or beverage as recited in claim 3, wherein pectin is apple-derived or citrus-derived pectin.
